# EUROPEAN PATENT APPLICATION

(11) **EP 1 424 389 A1**
(43) Date of publication of application: **02.06.2004**
(21) Application number: 02760579.9
(22) Date of filing: 07.08.2002
(51) Int. Cl.: C12N 5/06, C12N 5/08, C12N 5/02

(54) **PROCESS FOR PREPARING HEMATOPOIETIC STEM CELLS**

(30) Priority: 07.08.2001 JP 2001239576
(71) Applicant: KIRIN BEER KABUSHIKI KAISHA, Tokyo 104-8288 (JP); Tosoh Corporation, Shunan-shi Yamaguchi-ken 746-0006 (JP)
(72) Inventor: NISHIKAWA, Mitsuo, c/o Kirin Beer K. K., Takasaki-shi, Gunma 370-1295 (JP); OSAWA, Masatake, Ashiya-shi, Hyogo 659-0051 (JP); ISHIGURO, Takahiko, Yokohama-shi, Kanagawa 222-0034 (JP); YASUKAWA, Kiyoshi, Kawasaki-shi, Kanagawa 215-0021 (JP)
(74) Representative: HOFFMANN - EITLE
(86) International application number: PCT/JP2002/008087
(87) International publication number: WO 2003/014336

(57) **Abstract**

An objective of the present invention is to provide a process for effectively producing hematopoietic stem cells. A process for producing hematopoietic. stem cells comprises the step of culturing hematopoietic stem cells in the presence of a gp130 stimulating factor, one or more cytokines and stromal cells.

## Description

### BACKGROUND OF THE INVENTION

### Field of the Invention

The present invention relates to process for producing hematopoietic stem cells and a medium used for culturing hematopoietic stem cells.

### Background Technology

The life span of matured blood cells in the body is short and homeostasis of the blood is maintained by replenishing matured blood cells through continuous differentiation of hematopoietic progenitor cells. The hematopoietic progenitor cells are generated by differentiation of more undifferentiated hematopoietic stem cells. The hematopoietic stem cells have an ability to differentiate into various types. of lineages (multidifferentiation potential) and can supply hematopoietic cells throughout life by self-renewing while maintaining their multidifferentiation potential. Namely, hematopoietic stem cells are known to generate stem cells having the multidifferentiation potential by self-renewing while they partly differentiate into various types of matured blood cells via hematopoietic progenitor cells.

Such differentiation into blood cells is regulated by various kinds of cytokines. It is known that erythropoietin (EPO), granulocyte colony stimulating factor (G-CSF), and thrombopoietin (TPO) enhance differentiation of cells of erythrocyte lineage, cells of basophilic myeloid lineage, and magakaryocytes and platelet-producing cells, respectively. However, it is not known what kind of factors is required for hematopoietic stem cells to self-renew while maintaining multidifferentiation potential. Several factors have been reported as growth factors for hematopoietic stem cells, which include SCF/MGF (Williams, D. E. Cell 63:167-174, 1990; Zsebo, K. M. Cell 63:213-224, 1990) and SCGF (W098/08869); however none of the factors can sufficiently maintain the multidifferentiation potential. Further, there have been attempts to culture hematopoietic stem cells by adding multiple kinds of known cytokines in combination (Miller CL. Proc. Natl. Acad. Sci. USA 94:13648-13653, 1997; Yagi M. Proc. Natl. Acad. Sci. USA 96: 8126-8131, 1999; Shih C. C. Blood 94:5 1623-1636, 1999).

On the other hand, there have been attempts to utilize stromal cells which provide an suitable environment for the maintenance and expansion of hematopoietic cells to support the maintenance and expansion of hematopoietic stem cells without differentiation (Moore K. A. Blood 89:12 4337-4347,.1997, Expansion in vitro of adult murine hematopoietic stem cells with transplantable lympho-myeloid reconstituting ability, Cindy L. Miller and Connie J. Eaves, PNAS 94:13648-13653). Also, WO99/03980 discloses a stromal cell line cloned from the AGM (aorta-gonad-mesonephros) region of a murine fetus, which can support the expansion or survival of hematopoietic stem cells and hematopoietic progenitor cells. Further, culture using SCF, FL, TPO, sIL-6R-IL-6 fusion protein (Hyper-IL-6) and stromal cells has been described in Rappold I, Watt SM, Kusadasi N, Rose-John S, Hatzfeld J, Plosmacher RE. Gp130-signaling synergizes with FL and TPO for the long-term expansion of cord blood progenitors, Leukemia, 1999 Dec, 13(12): 2036-48.

However, the culture system of Rappold et al. is a culture system for hematopoietic progenitor cells and entirely different from a culture system for hematopoietic stem cells. Furthermore, Kusadasi N et al. (Leukemia. 2000, 11:1944-1953) has reported that the function of stromal cells is compensated by F1t-3-ligand (FL) + TPO in the culture system of IL-6, FL and TPO and the stromal cells has little activity in enhancing the function in stem cell maintenance.

### SUMMARY OF THE INVENTION

Recently, the present inventors found that the expansion and survival of hematopoietic stem cells can be effectively supported by culturing the hematopoietic stem cells in the presence of gp130 stimulating factor, a cytokine cocktail, and stromal cells.

An object of the present invention is to provide an effective process for producing hematopoietic stem cells, an effective method for culturing hematopoietic stem cells, and a medium for use in culturing hematopoietic stem cells.

A. process for producing hematopoietic stem cells according to the present invention comprises the step of culturing hematopoietic stem cells in the presence of gp130 stimulating factor, one or more cytokines, and stromal cells.

A method for culturing hematopoietic stem cells according to the present invention comprises the step of culturing hematopoietic stem cells in the presence of gp130 stimulating factor, one or more cytokines, and stromal cells.

A medium for use in culturing hematopoietic stem cells according to the present invention comprises gp130 stimulating factor, one or more cytokines, and stromal cells.

In hematopoietic stem cell transplantation therapy, such as peripheral blood stem cell transplantation and umbilical cord blood stem cell transplantation, transplantation is not feasible because a sufficient number of hematopoietic stem cells for transplantation cannot be obtained. According to the present invention, hematopoietic stem cells can be efficiently expanded in vitro and can be stably maintained. Therefore, even when a sufficient number of hematopoietic stem cells cannot be collected from a patient, the necessary amount of hematopoietic stem cells can be cultured and transplanted into the patient. Namely, the present invention paves the way for practical use of therapies which require blood cell transplantation and transplantations in general.

Also, hematopoietic stem cells expanded by a culture system according to the present invention can be used as cells to be differentiated into tissues other than blood. Therefore, hematopoietic stem cells obtained according to the present invention can be used for the regeneration of normal cells in treating myocardial necrosis caused by hepatic failure, muscular dystrophy, myocardial infarction and the like and vascular necrosis caused by diabetes, intestinal tract cell necrosis, or for therapeutic regeneration of nerve cells.

### BRIEF DESCRIPTION OF THE DRAWINGS

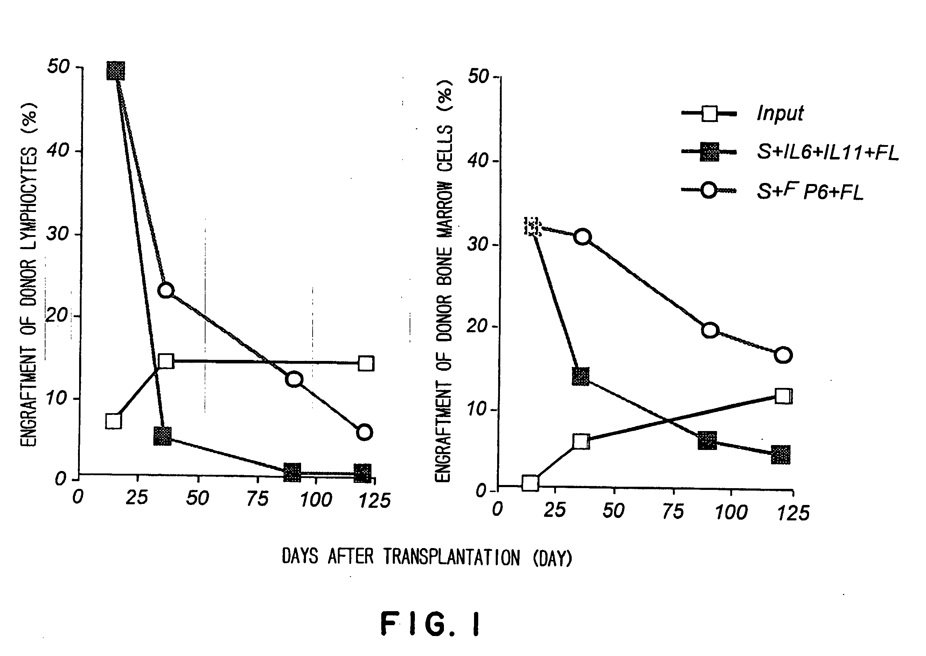

Figure 1 shows the effect of cytokine cocktails on the expansion of hematopoietic stem cells, demonstrating the change with time in chimerism of donor-derived blood cells in peripheral blood of an individual transplanted with murine hematopoietic stem cells. "Input" (□) is the case where murine hematopoietic stem cells were transplanted immediately after harvesting into an irradiated mouse. "S + IL6 + IL11 + FL" (black square) is the case where murine hematopoietic stem cells were harvested, cultured in the presence of cytokines, SCF + IL-6 + IL-11 + FL, and then transplanted into an irradiated mouse. "S + FP6 + FL" (O)is the case where murine hematopoietic stem cells were harvested, cultured in the presence of cytokines, SCF + FP6 + FL, and then transplanted into an irradiated mouse.

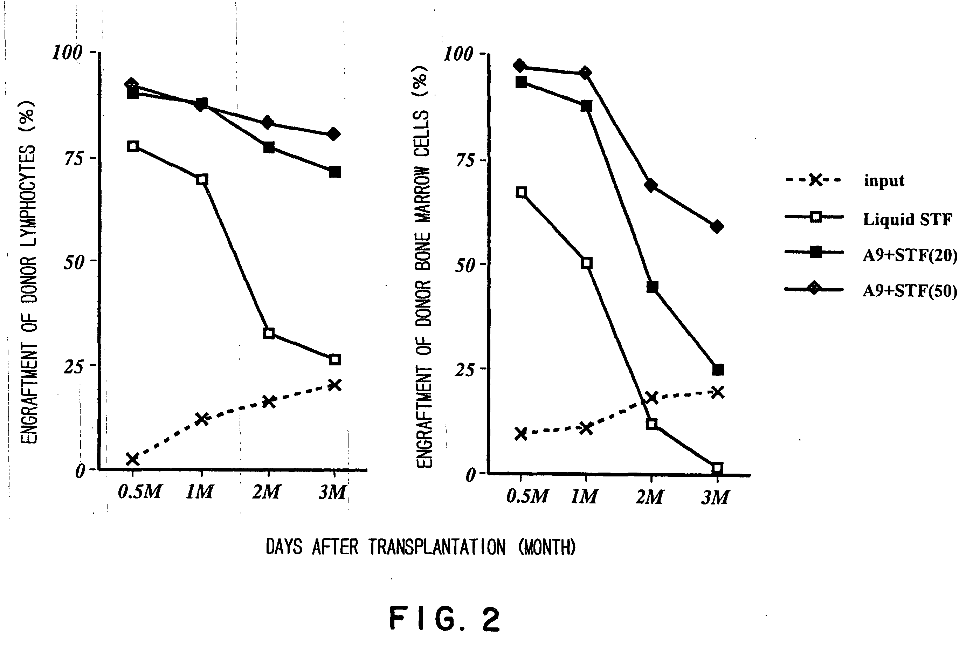

Figure 2 shows the effect of cytokine cocktails on the expansion of hematopoietic stem cells, demonstrating the change with time in chimerism of donor-derived blood cells in peripheral blood of an individual transplanted with murine hematopoietic stem cells. "Input" (×) is the case where murine hematopoietic stem cells were transplanted immediately after harvesting into an irradiated mouse. "Liquid STF" (□) is the case where murine hematopoietic stem cells were harvested, cultured in the presence of cytokines, SCF + FP6 + TPO, and then transplanted into an irradiated mouse. "A9 + STF" (black square and ◆) is the case where murine hematopoietic stem cells were harvested, cocultured with AGM-S3-A9 stromal cells in the presence of cytokines, SCF + FP6 + TPO, and then transplanted into an irradiated mouse. "A9 + STF (20)" (black square) shows the result when the FP6 concentration was 20 ng/ml. "A9 + STF (50)" (◆) shows the result when the FP6 concentration was 50 ng/ml.

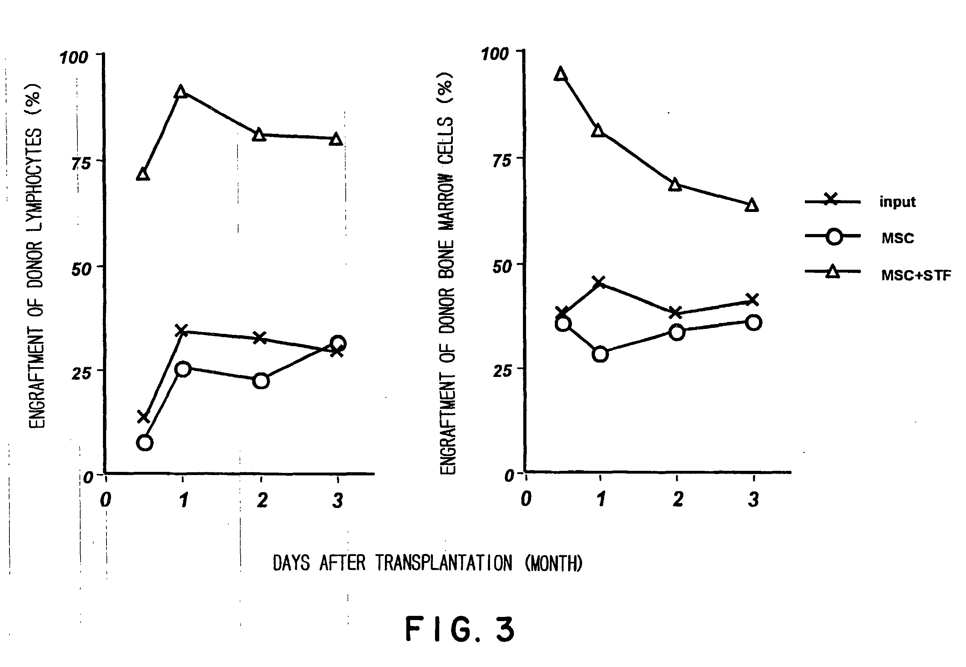

Figure 3 shows the effect of human mesenchymal stem cells and cytokine cocktails on the expansion of hematopoietic stem cells. More specifically, the Figure shows the change with time in chimerism of donor-derived blood cells in peripheral blood of an individual transplanted with murine hematopoietic stem cells. "Input" (×) is the case where murine hematopoietic stem cells are transplanted into an irradiated mouse immediately after harvesting. "MSC" (○) is the case where murine hematopoietic stem cells were harvested, cocultured with human mesenchymal stem cells, and then transplanted into an irradiated mouse. "MSC + STF" (Δ) is the case where murine hematopoietic stem cells were harvested, cocultured with human mesenchymal stem cells in the presence of cytokines, SCF + FP6 + TPO, and then transplanted into an irradiated-mouse.

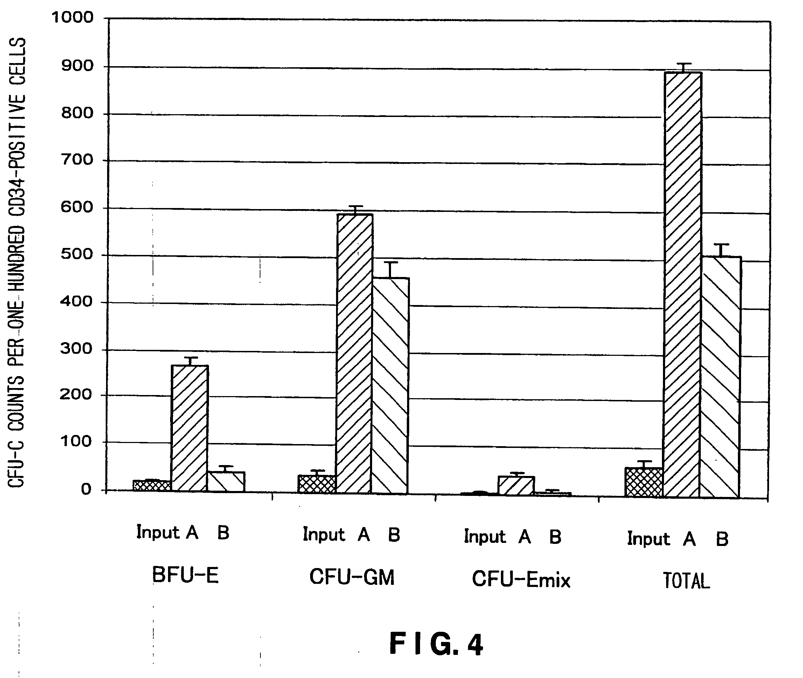

Figure 4 shows the effect of stromal cells and cytokine cocktails on the expansion of hematopoietic stem cells and hematopoietic progenitor cells. "Input" is the case where human hematopoietic stem cells were subjected to colony assay immediately after harvesting. "A" is the case where human hematopoietic stem cells were harvested, cocultured with AGM-S3-A9 stromal cells in the presence of cytokines, SCF + FP6 + TPO + FL, and then subjected to colony assay. "B" is the case where harvested human hematopoietic stem cells are cultured in the presence of cytokines, SCF + FP6 + TPO + FL, and then subjected to colony assay. "BFU-E": BFU-E (erythroid burst-forming unit), "CFU-GM": CFU-GM (granulocyte-macrophage colony-forming unit), "CFU-Emix": CFU-Emix (erythrocyte mixed colony-forming unit).

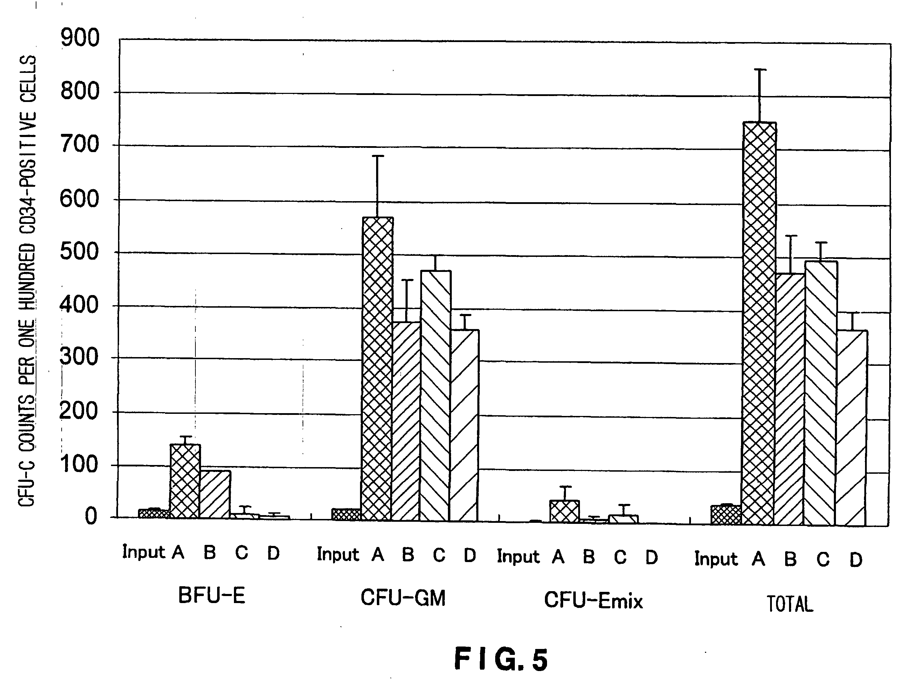

Figure 5 shows the effect of stromal cells and cytokine cocktails on the expansion of hematopoietic stem cells and hematopoietic progenitor cells. "Input" is the case where human hematopoietic stem cells were subjected to colony assay immediately after harvesting. "A" is the case where human hematopoietic stem cells were harvested, cocultured with AGM-S3-A9 stromal cells in the presence of cytokines, SCF + FP6 + TPO + FL, and then subjected to colony assay. "B" is the case where human hematopoietic stem cells were harvested, cocultured with AGM-S3-A9 stromal cells in the presence of cytokines, SCF + TPO + FL, and then subjected to colony assay. "C" is the case where human hematopoietic stem cells were harvested, cultured in the presence of cytokines, SCF + FP6 + TPO + FL, and then subjected to colony assay. "D" is the case where human hematopoietic stem cells were harvested, cultured in the presence of cytokines, SCF + TPO + FL, and then subjected to colony assay. "BFU-E": BFU-E (erythroid burst-forming unit), "CFU-GM": CFU-GM (granulocyte-macrophage colony-forming unit), "CFU-Emix" : CFU-Emix (erythrocyte mixed colony-forming unit).

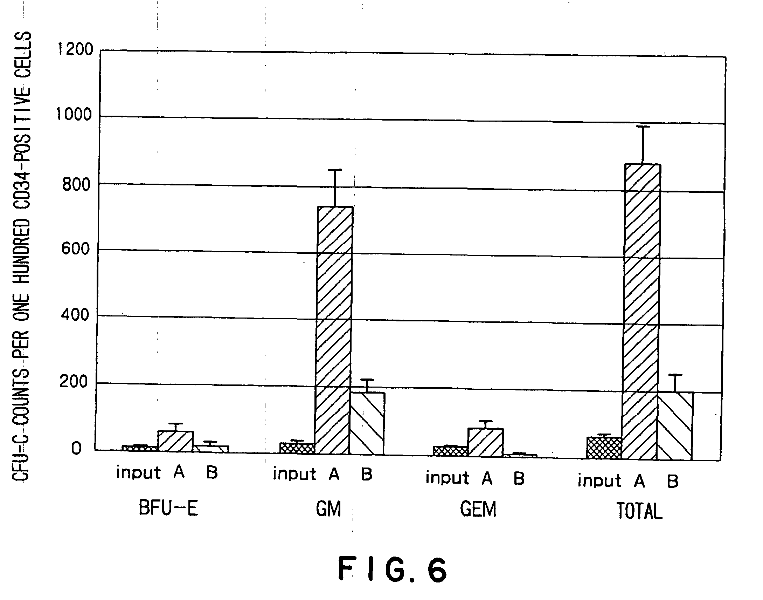

Figure 6 shows the effect of stromal cells and cytokine cocktails on the expansion of hematopoietic stem cells and hematopoietic progenitor cells. "Input" is the case where human hematopoietic stem cells were subjected to colony assay immediately after harvesting. "A" is the case where human hematopoietic stem cells were harvested, cocultured with AGM-S3-A9 stromal cells in the presence of cytokines, SCF + FP6 + TPO + FL, and then subjected to colony assay. "B" is the case where human hematopoietic stem cells were harvested, cocultured with AGM-S3-A9 stromal cells in the presence of cytokines, SCF + FP6 + FL, and then subjected to colony assay. "BFU-E": BFU-E (erythroid burst-forming unit), "CFU-GM": CFU-GM (granulocyte-macrophage colony-forming unit), "CFU-Emix": CFU-Emix (erythrocyte mixed colony-forming unit) .

### DETAILED DESCRIPTION OF THE INVENTION

### Hematopoietic stem cells

In the present invention, the term "hematopoietic stem cell" refers to a cell having the ability to differentiate into all lineages of the blood cells (multidifferentiation potential) and the self-renewability while maintaining its multidifferentiation potential.

Hematopoietic stem cells can be harvested from body materials of mammals such as humans and mice, including the fetal liver, bone marrow, fetal bone marrow, peripheral blood, peripheral blood into which stem cells are mobilized by administration of cytokines and/or anticancer agents, and umbilical cord blood.

In mice, hematopoietic stem cells and hematopoietic progenitor cells can be distinguished by a transplantation experiment. Namely, if hematopoietic cells of differentiation lineage of both donor-derived bone marrow cells and lymphocytes are found in an irradiated mouse (recipient) transplanted with test cells for more than three months after the transplantation, it means that hematopoietic stem cells having the multidifferentiation ability and self-renewability by the recipient itself were present in the transplanted cell population.

Erythrocyte progenitor cells, which are difficult to be maintained and expanded, quickly disappear in an in vitro culture environment. Presumably, in the case where the maintenance and expansion of erythrocyte progenitor cells are confirmed, more undifferentiated hematopoietic stem cells or hematopoietic progenitor cells are maintained and expanded, continuously producing erythrocyte progenitor cells. Therefore, in an evaluation system for human hematopoietic stem cells, the expansion of hematopoietic stem cells or undifferentiated hematopoietic progenitor cells can be confirmed by evaluating the maintenance and expansion of erythrocyte progenitor cells (BFU-E) or progenitor cells differentiable into two cell lineages, granulocyte and macrophage (CFU-GM). Further, the presence of hematopoietic stem cells or hematopoietic progenitor cells in the transplanted cell population can be confirmed by transplanting human hematopoietic cells to an immune deficient mouse NOD/SCID and evaluating the frequency of the presence of human hematopoietic cells after the transplantation.

### gp130 stimulating factor

In the present invention, the term "gp130 stimulating factor" refers to a molecule transducing signals through human gp130, such as a complex of IL-6 with IL-6 receptor α-chain (hereinafter referred to as "IL-6R"), namely a molecule which activates gp130 by associating with gp130, and then causes activation of JAK kinase, phosphorylation of STAT3, and enhancement of transcription of genes such as JunB, JAB, SAA3, and C-reactive protein by STAT3, thereby generating cell growth, cell differentiation regulation, and the like. For assessing gp130 stimulating factors, IL-3-dependent murine cell line Ba/F3, which originally does not express gp130, is transformed to express a gene encoding human gp130, and the growth of this transformant can be detected since it grows by gp130 signal. The growth of the transformant can be confirmed, for example, by incorporating isotope-labeled nucleic acids into the cells, evaluating mitochondrial activity, or directly counting cell numbers; for example it can be carried out as described in Example 5 of Japanese Patent Laid-Open Publication No. 8690/2001. Further, since gp130 stimulation also activates STAT3, an intracellular signal transducing molecule, as described above, detection of STAT3 activation in the above-mentioned Ba/F3 cell can also be used to assess whether a substance of interest is gp130 stimulating factor or not. The STAT3 activation can be confirmed by detecting phophorylation of intracellular STAT3, or alternatively by detecting enhancement of transcription of the gene group, whose transcription is enhanced by STAT3, using the Northern analysis or quantitative RT-PCR method.

Examples of the gp130 stimulating factor include a fusion protein of IL-6 or its mutant with IL-6R or its mutant, a combination of IL-6R (preferably soluble IL-6R (sIL-6R) or its mutant and IL-6 or its mutant (Japanese Patent Laid-Open Publication No. 509040/1998), anti-gp130 antibody acting as an agonist against gp130 (Gu ZJ, Vos JD, Rebouissou C, Jourdan M, Zhang XG, Rossi JF, Wijdenes J, Klein B. Agonist anti-gp130 transducer monoclonal antibodies are human myeloma cell survival and growth factors, Leukemia, 2000 Jan, 14(1):188-97), interleukin-11 (IL-11), leukocyte migration inhibitory factor (LIF), oncostatin M, and cardiotropin.

In the present invention, the term "IL-6" refers to a protein having 212 amino acid residues consisting of four helices (Hirano et al. Nature 324, 731 (1986)). Further, in the present invention, the term "IL-6 mutant" refers to an IL-6 mutant having one or more modifications selected from a substitution, deletion, insertion, and addition and having gp130 stimulating activity. As to IL-6, it is known that gp130 stimulating activity is maintained even when a signal peptide, i.e., sequence from the N-terminal to alanine at residue 28 (WO00/01731). Further, it is known that the C-terminal side of lysine at residue 37 of IL-6 can be digested by protease and IL-6 activity can be maintained even when 10 amino acid residues from alanine at residue 28 to lysine at residue 37 are deleted (WO00/01731). Accordingly, in the present invention, a modified IL-6 which undergoes an N-terminal deletion and has gp130 stimulating activity can be used as an IL-6 mutant. Examples of such IL-6 mutant include IL-6 of which the N-terminal sequence up to residue 28 has been deleted and IL-6 of which the N-terminal sequence up to residue 37 has been deleted.

In the present invention, the term "IL-6R" refers to a protein having a length of 468 amino acid residues consisting of a signal region, extracellular region, transmembrane region, and intracellular region (Yamasaki et al. Science 241, p825 (1988)). Further, in the present invention, the term "IL-6R mutant" refers to an IL-6R mutant having one or more modifications selected from a substitution, deletion, insertion, and addition and having gp130 stimulating activity. As to IL-6R, a cytokine receptor region present in the extracellular region (a region from the vicinity of valine at residue 112 to the vicinity of alanine at residue 323) is known to be necessary and sufficient for the binding with IL-6 (Yawata et al. EMBO J. 12, p1705 (1993) ) . Accordingly, in the present invention, a modified IL-6R comprising a region from the vicinity of valine at residue 112 to the vicinity of alanine at residue 333 of IL-6R can be used as an IL-6R mutant.

The fusion protein is a protein composed of IL-6 or its mutant linked to IL-6R or its mutant, directly or via a linker. The fusion protein can be produced, for example, according to the method described in WO00/01731, WO99/02552, Japanese Patent Laid-Open Publication No. 506014/2000, or Fisher et al. Nature, Biotech. 15, p142 (1997).

The fusion protein can be a protein (FP6) composed of a fragment from valine at residue 112 to alanine at residue 333 of IL-6R directly linked at its C-terminal to the N-terminal of a fragment from aspartic acid at residue 38 to methionine at residue 212 of IL-6. In this case, the fusion protein FP6 can be produced by introducing a vector, into which a DNA sequence encoding the fusion protein is ligated to be expressed in a host, into an appropriate host, culturing the transformed host cells, and recovering FP6 from the culture (see WO00/01731).

### Cytokines

"Cytokines" used in the present invention can expand hematopoietic stem cells and maintain hematopoietic stem cells.

Examples of such cytokines include growth factors such as thrombopoietin (TPO) and mutants thereof and their derivatives, c-mpl ligands (excluding TPO) and derivatives thereof, stem cell factor (SCF), Flt-3 ligand (FL), IL-3 (interleukin-3) , granulocyte-macrophage colony stimulating factor (GM-CSF), interleukin-6 (IL-6), granulocyte colony stimulating factor (G-CSF), transforming growth factor-β (TGF-β), and MIP-1α (Davatelis, G. J. Exp. Med. 167:1939, 1988); hematopoietic hormones such as erythropoietin (EPO); differential growth regulatory factors such as chemokines, Wnt gene products, and Notch ligands; embryonic regulatory factors; and combinations thereof.

In the present invention, "thrombopoietin (TPO)" is a protein consisting of an amino acid sequence of 332 amino acid residues and having activity to specifically stimulate or augment platelet production (WO95/21919). Further, in the present invention, the term "TPO mutant" refers to a TPO mutant which has one or more modifications selected from a substitution, deletion, insertion, and addition and specifically stimulates or enhances platelet production. Examples of the TPO mutant include those described in WO95/18858, Japanese Patent No. 2991640, Japanese Patent No. 2991630, and Japanese patent No. 2996729, and preferably, a TPO mutant consisting of the amino acid sequence from amino acid residue 1 to 163.

In the present invention, the term "derivative of TPO and its mutant" refers to TPO or its mutant ligated to an aqueous polymer. Examples of the aqueous polymer include polyethylene glycols, preferably polyethylene glycols having an average molecular weight of about 5 kDa to about 50 kDa. Ligation of TPO and its mutant to a polyethylene glycol can be carried out by a known method (for example, see Focus on Growth Factors 3(2):4-10 (1992)). The molar ratio of aqueous polymer to c-mpl ligand protein can be from 1:1 to 100:1; from 1:1 to 20:1 for poly PEGS, and from 1:1 to 5:1 for mono PEGS.

In the present invention, the term "c-mpl ligand" refers to a peptide ligand, protein ligand, and non-peptide ligand that can bind to an mpl receptor. Examples of the protein c-mpl ligand (excluding TPO) include an agonistic antibodies as megakaryocyte stimulating agents (WO99/03495, W099/10494) and other proteins such as hematopoietic receptor agonists (WO96/23888, WO97/12978, WO97/12985, WO98/17810, WO96/34016, WO00/24770). Examples of the peptide c-mpl ligand include those described in WO96/40189, WO96/40750, WO98/25965, Japanese Patent Laid-Open Publication No. 72492/1998, WO99/42127, and WO00/24770. Examples of the non-peptide c-mpl ligand include benzodiazepin derivatives (Japanese Patent Laid-Open Publication No. 1477/1999, Japanese Patent Laid-Open Publication No. 152276/1999) and other low molecular weight ligands (WO99/11262, WO99/22733, WO99/22734, WO00/35446, WO00/28987).

In the present invention, examples of preferred cytokines include stem cell factor (SCF); thrombopoietin (TPO) and its mutants and derivatives thereof; Flt-3 ligand (FL); and combinations thereof.

In the present invention, examples of preferred cytokines include those at least containing thrombopoietin (TPO) or its mutants or derivatives thereof.

### Stromal cells

In the present invention, the term "stromal cell." refers to a cell which can provide a hematopoietic environment in vitro equivalent or similar to the hematopoietic environment in the body and can be from any origin as far as it is co-culturable with hematopoietic cells in vitro.

Examples of the stromal cell include cells derived from the AGM (aorta-gonad-mesonephros) region, bone marrow-derived stromal cells, mesenchymal stem cells, fibroblasts, vascularendothelial cells, fetal liver-derived cells, and preadipocytes, preferably the cells derived from the AGM region, bone marrow-derived stromal cells, and mesenchymal stem cells, and most preferably the cells derived from the AGM region and mesenchymal stem cells.

Alternatively, a recombinant vector in which a suicide gene or the like -is inserted downstream of the promoter capable of artificially regulating expression, is introduced into a stromal cell, after culturing with hematopoietic stem cells, only stromal cells are killed, and then stromal cells can be removed upon transplantation. Introduction and expression of the suicide gene can be carried out as follows. Namely, the suicide gene can be expressed by suicide gene expression technique, more specifically by using a diphtheria toxin (Lidor YJ, Lee WE, Nilson JH, Maxwell IH, Su LJ, Brand E, Glode LM. In vitro expression of the diphtheria toxin A-chain gene, under the control of human chorionic gonadotropin gene promoters as a means of directing toxicity to ovarian cancer cell lines. Am J Obstet Gynecol. 1997 Sep, 177(3):579-85), by using herpes virus thymidine kinase (HStk) (Link CJ, Seregina T, Traynor A, Burt RK. Cellular suicide therapy of malignant disease. Oncologist 5(1):68-74, 2000), or by expressing apoptosis-related genes. Examples of the apoptosis-related genes to be used include receptor genes having a death domains such as Fas (Itoh N. Cell 66:233-243, 1991), TNF receptor type II (Loetscher H. Cell 61 (2): 351-359, 1990), Death receptor 3 (Kitoson J. Nature 384:372-375, 1996), and Death receptor 4 (Pan G. Science 276:111-113, 1997), Flice which is a signal transducing molecule of the abovementioned receptor, ICE (IL-1 b converting enzyme) which is a proteinase belonging to a signal transduction system (Cerretti D.P. Science 256:97-100, 1992), and further the caspase family (Patel T. FASEB. J. 10:587-597, 1996) such as CPP32 (Fernandes-Alnemri T. J. Biol. Chem. 269:30761-30764, 1994). Further, in order to artificially regulate expression of these genes, an expression induction system using tetracycline can be utilized (Manfred G. Proc. Natl. Acad. Sci. USA. 89:5547-5551, 1992). In order to introduce these genes into stromal cells, methods generally used for introducing genes into animal cells can be used except that a stromal cell line is used as a host. Examples of such methods include a method using a virus-derived vector for animal cells, such as a retrovirus (e.g., Moloney murine leukemia virus) vector, adenovirus vector, adeno-associated virus (AAV) vector (Kotin, R.M. Hum Gene Ther 5, 793 (1994)), and herpes simplex virus vector, a calcium phosphate precipitation method, a DEAE-dextran method, an electroporation method, a liposome method, a lipofection method, and a microinjection method. In introducing a suicide-related gene into a stromal cell, a marker gene such as a drug-resistance gene can be used in addition to said gene to facilitate the selection of the stromal cells into which the target gene has been introduced.

### Culture

According to the present invention, there is provided a culture system to expand hematopoietic stem cells. By using the culture system, the growth and survival of hematopoietic stem cells can be effectively maintained.

A culture system according to the present invention contains hematopoietic stem cells, and may contain other hematopoietic cells. Further, it may be a fraction containing hematopoietic stem cells. Namely, in the system, it is possible to culture a fraction which is prepared by isolating hematopoietic stem cells from umbilical cord blood, peripheral blood, or tissues containing hematopoietic stem cells such as bone marrow using an antibody which specifically recognizes hematopoietic stem cells. Various kinds of cells such as erythrocytes can be specifically removed by a conventional blood cell fractionation method. Cultivation may be carried out without fractionation.

In culturing hematopoietic stem cells, a conventional culture system using petri dishes, flasks, or culture bags can be used; however, the system can be improved by using a bioreactor which supports high density culture by mechanically controlling medium composition, pH and the like (Schwartz, Proc. Natl. Acad. Sci. USA. 88:6760,1991; Koller, M.R. Bio/Technology 11:358, 1993; Koller, M.R. Blood 82: 378, 1993; Palsson, B.O. Bio/Technology 11:368, 1993).

Cocultivation of stromal cells with hematopoietic cells can be carried out by culturing cells as they are after harvesting bone marrow. It is also possible to isolate stromal cells, hematopoietic cells, and other cell groups from the harvested bone marrow and cocultivate in combination with stromal cells and hematopoietic cells of an individual other than the one from which the bone marrow has been harvested. It is also possible to culture stromal cells alone for growth and then carry out cocultivation with addition of a cytokine cocktail and hematopoietic cells. In culturing stromal cells, a cell stimulating factor can be added to the culture system to support the growth and survival more effectively. Examples of such cell stimulating factor include growth factors represented by cytokines such as thrombopoietin (TPO). and its mutants and derivatives thereof, c-mpl ligands (excluding TPO) and derivatives thereof, stem cell factor (SCF), Flt-3 ligand (FL), interleukin-3 (IL-3), granulocyte-macrophage colony stimulating factor (GM-CSF), interleukin-6 (IL-6), granulocyte colony stimulating factor (G-CSF), transforming growth factor-β (TGF-β), and MIP-1α (Davatelis, G. J. Exp. Med. 167:1939, 1988); hematopoietic hormones such as erythropoietin (EPO); differential growth regulatory factors such as chemokines, Wnt gene products, and Notch ligands; and embryonic regulatory factors. These cytokines can be produced by stromal cells. Any substance that transduces a signal similar to cytokines via a cytokine receptor can be used similarly to these cytokines.

A medium to be used for cultivation is not particularly limited as long as it does not interfere with the growth and survival of hematopoietic stem cells. Preferred examples include MEM-α medium (GIBCO BRL), SF-02 medium (Sanko Pure Chemicals), Opti-MEM medium (GIBCO BRL), IMDM medium (GIBCO BRL), and PRMI 1640 medium (GIBCO BRL). The culture temperature is generally 25°C to 39°C, preferably 33°C to 39°C. Further, examples of substances to be added include fetal calf serum, human serum, horse serum, insulin, transferin, lactferin, ethanolamine, sodium selenite, monothioglycerol, 2-mercaptoethanol, bovine serum albumin, sodium pyruvate, polyethylene glycol, various vitamins, and various amino acids. The CO₂ concentration is generally 4% to 6%, preferably 5%.

Since hematopoietic stem cells can be differentiated into all lineages of hematopoietic systems, it is possible to expand hematopoietic stem cells in vitro and then differentiate them into various kinds of cells to transplant. For example, when erythrocytes are needed, hematopoietic cells having erythrocytes as a major component can be artificially produced by expanding patient's stem cells by cultivation and then adding an agent to enhance induction of erythrocyte differentiation, such as EPO.

A method according to the present invention may include the step of harvesting hematopoietic stem cells from the culture after cultivation of the hematopoietic stem cells.

Hematopoietic stem cells cultured by a method according to the present invention can be detached from a culture vessel using a substance used as a cell dispersing solution such as an EDTA solution or trypsin solution containing EDTA to prepare a cell suspension administrable to the human body.

Hematopoietic stem cells cultured by a method according to the present invention can be separated into hematopoietic stem cells and stromal cells and then transplanted into a patient. It is also possible to simultaneously transplant the stromal cells and hematopoietic stem cells.

Among methods according to the present invention, preferred is a method comprising the step of culturing hematopoietic stem cells in the presence of a fusion protein of interleukin-6 (IL-6) with IL-6 receptor α-chain (IL-6R), thrombopoietin (TPO), and stromal cells selected from AGM region-derived cells, bone marrow-derived stromal cells, and mesenchymal cells, and optionally, a stem cell factor (SCF) and an Flt-3 ligand (FL).

Among the media according to the present invention, preferred is a medium comprising a fusion protein of interleukin-6 (IL-6) with IL-6 receptor α-chain (IL-6R), thrombopoietin (TPO), and stromal cells selected from AGM region-derived cells, bone marrow-derived stromal cells, and mesenchymal cells, and optionally, a stem cell factor (SCF) and an Flt-3 ligand (FL).

### Use of hematopoietic stem cells

Hematopoietic stem cells cultured using a culture system according to the present invention can be used as a graft for transplantation of blood cells in place of conventional bone marrow transplantation or umbilical cord blood transplantation. Since a graft in hematopoietic stem cell transplantation can be engrafted semipermanently, conventional blood cell transplantation therapy can be improved.

Transplantation of hematopoietic stem cells can be carried out in systemic X-ray radiotherapy or high dose chemotherapy for leukemia. For example, in a therapy such as chemotherapy and radiotherapy for a solid cancer patient, which causes bone marrow suppression as a side effect, bone marrow is harvested prior to the therapy and hematopoietic stem cells are expanded in vitro and then returned to the patient after the therapy; in this way, the disorder of hematopoietic system caused by the side effect can be ameliorated in early stages, more powerful chemotherapy can be carried out, and therapeutic effect of the chemotherapy can be improved. Further, patient's dysfunctional conditions caused by the suppressed production of blood cells can be improved by differentiating hematopoietic stem cells obtained according to the present invention into various blood cells and then transplanting them into the patient's body. Further, hematopoietic insufficiency associated with anemia such as aplastic anemia caused by bone marrow hypoplasia can be improved. Examples of other diseases for which hematopoietic stem cells transplantation by a method according to the present invention is effective include immune deficiency syndromes such as chronic granuloma, multiple immune deficiency syndrome, agammaglobulin anemia, Wiskott-Aldrich syndrome, and acquired immune deficiency syndrome (AIDS); thalassemia; hemolytic anemia caused by oxygen depletion; congenital anemia such as sickle cell anemia; diseases associated with lysosomal accumulation such as Gaucher's disease and mucopolysaccharide disease; adenoleukodystrophy; and various cancers and tumors.

Transplantation of hematopoietic stem cells can be carried out in the same manner as conventional bone marrow transplantation and umbilical cord blood transplantation, except for the cells to be used.

The origin of hematopoietic stem cells to be used for the abovementioned hematopoietic stem cell transplantation is not limited to bone marrow, and the abovementioned fetal liver, bone marrow, fetal bone marrow, peripheral blood, peripheral blood into which stem cells are mobilized by administered cytokines and/or anticancer agents, umbilical cord blood, and the like can be used.

A graft can be formulated as a composition containing a buffer solution and the like in addition to hematopoietic stem cells produced according to a method of the present invention.

Further, hematopoietic stem cells produced according to the present invention can be used in ex vivo gene therapy. Gene therapy on hematopoietic stem cells was difficult mainly because stem cells are in the stationary phase, in which the rate of recombination with chromosome is low, and eventually differentiated during culture. According to the present invention, stem cells can be expanded without being differentiated, and the efficiency of gene introduction can be highly improved. This gene therapy is carried out by introducing a foreign gene (therapeutic gene) into hematopoietic stem cells and using the resulting cells carrying the introduced gene. The foreign gene to be introduced is appropriately selected depending on the disease. Examples of the diseases subjected to the gene therapy, in which blood cells are targeted, include immune deficiency syndromes such as chronic granuloma, multiple immune deficiency syndrome, agammaglobulin anemia, Wiskott-Aldrich syndrome, and acquired immune deficiency syndrome (AIDS); thalassemia; hemolytic anemia caused by oxygen depletion; congenital anemia such as sickle cell anemia; diseases associated with lysosomal accumulation such as Gaucher's disease. and mucopolysaccharide disease; adenoleukodystrophy; and various cancers and tumors.

A therapeutic gene can be introduced into a hematopoietic stem cell by a method generally used for gene introduction for animal cells, including a method using a virus-derived vector for animal cells usable in gene therapy, for example, a retrovirus vector such as Moloney murine leukemia virus vector, adenovirus vector, adeno-associated virus (AAV) vector, herpes simplex virus vector, HIV vector, and feline endogenous virus vector RD114 (Kelly PF, Vandergriff J, Nathwani A, Nienhuis AW, Vanin EF. Highly efficient gene transfer into cord blood nonobese diabetic/severe combined immunodeficiency repopulating cells by oncoretroviral vector particles pseudotyped with the feline endogenous retrovirus (RD114) envelope protein. Blood. 2000 Aug 15, 96(4):1206-14) (see Verma, I.M. Nature 389:239, 1997 for vectors for gene therapy), a calcium phosphate precipitation method, a DEAE-dextran method, an electroporation method, a liposome method, a lipofection method, and a microinjection method. Among them, use of a retrovirus vector, adeno-associated virus vector or HIV vector is preferable in terms that a gene is incorporated into chromosomal DNA of a target cell and thus perpetual gene expression can be expected.

For example, an adeno-associated virus (AAV) vector can be constructed as follows. First, 293 cells are. transfected with a vector plasmid having a therapeutic gene inserted between ITRs (inverted terminal repeats) on both ends of the wild-type adeno-associated viral DNA and a helper plasmid to compensate viral protein. The cells are then infected with a helper virus, i.e., adenovirus, to produce viral particles containing the AAV vector. Alternatively, instead of adenovirus, a plasmid which expresses an adenovirus gene having a helper function can be used for transfection. Next, hematopoietic stem cells are infected with the resulting viral particles. In the vector DNA, it is preferable to regulate the gene expression by ligating an appropriate promoter, an appropriate terminator, and an enhancer upstream, downstream, and upstream or downstream of the target gene, respectively. Further, insulators can be ligated upstream and downstream of the target gene to block silencing. Further, in order to facilitate the selection of cells carrying the therapeutic gene introduced, a marker gene such as a drug resistance gene can be ligated in addition to the therapeutic gene. The therapeutic gene can be either a sense gene or an antisense gene.

The composition for gene therapy can be a composition further containing a buffer solution, novel active substances and the like in addition to hematopoietic stem cells produced by a method according to the present invention.

Further, it is possible to use hematopoietic stem cells expanded using a culture system according to the present invention as cells to be differentiated into tissues other than blood. Although stem cells present in various tissues in the body have conventionally been believed to function exclusively as stem cells of the individual tissues, recent reports have shown that they function also as stem cells of other organs. Hematopoietic stem cells or cell populations containing hematopoietic stem cells have been shown to have ability to differentiate into cells such as hepatic cells (Lagasse E, Connors H, Al-Dhalimy M, Reitsma M, Dohse M, Osborne L, Wang X, Finegold M, Weissman IL, Grompe M. Purified hematopoietic stem cells can differentiate into hepatocytes in vivo. Nat Med. 2000 Nov; 6(11):1229-34, [2127] PRODUCTION OF HUMAN HEPATOCYTES BY HUMAN LIN-, CD34+/- CELLS IN VIVO; Esmail D. Zanjani, Christopher D. Porada, Kirsten B. Crapnell, Neil D. Theise, Dianne S. Krause, F.R. MacKintosh, Joao L. Ascensao, Graca Almeida-Porada. Department of Veterans Affairs Medical Center, Reno, NV, USA, University of Nevada School of Medicine, Reno, NV, USA, New York University School of Medicine, New York, NY, USA, Yale University, New Haven, CT, USA. Blood 2000 96(11):494a), skeletal muscle cells (Gussoni E, Soneoka Y, Strickland CD, Buzney EA, Khan MK, Flint AF, Kunkel LM, Mulligan RC., Dystrophin expression in the mdx mouse restored by stem cell transplantation. Nature. 1999 Sep 23; 401(6751):390-4), myocardial cells (Orlic D, Kajstura J, Chimenti S, Jakoniuk I, Anderson SM, Li B, Pickel J, McKay R, Nadal-Ginard B, Bodine DM, Leri A, Anversa P. Bone marrow cells regenerate infarcted myocardium. Nature. 2001 Apr 5; 410:701-5), vascular endothelial cells, and intestinal mucous membrane cells (Krause DS, Theise ND, Collector MI, Henegariu O, Hwang S, Gardner R, Neutzel S, Sharkis SJ. Multi-organ, multi-lineage engraftment by a single bone marrow-derived stem cell. Cell. 2001 May 4; 105(3):369-77). Accordingly, hematopoietic stem cells produced by a culture method according to the present invention can be used for reproduction of normal cells in treating hepatic insufficiency, myodystrophy, cardiac necrosis caused by myocardial infarction, vascular necrosis caused by diabetes, or necrosis of intestinal membrane calls. Also, hematopoietic stem cells produced by a method according to the present invention can be used for nerve cell regeneration therapy.

### EXAMPLES

### Example 1: Preparation of hematopoietic stem cells from murine bone marrow

Bone marrow of thighbones from C57BL/6-Ly5.1 pep (8- to 10-week-old male) (provided by Prof. Hiromitsu Nakauchi, University of Tsukuba) was harvested and suspended in PBS. According to an ordinary method (Basic Technology in Immunological Research by Seiji Takatsu. Yodosha, 1995) , a murine bone marrow mononuclear cell fraction was concentrated by density gradient centrifugation, after which the concentrate was suspended in staining buffer (PBS containing 5% FCS and 0.05% NaN₃) to obtain a hematopoietic stem cell fraction according to the method of Osawa M. et al. Science 273:242-245, 1996, as follows.

To the bone marrow mononuclear cell suspension were added FITC-binding CD34 antibody, phycoerythrin-binding Sca-1 antibody, and allophycocyanin c-Kit (all from Pharmingen), and 6 kinds of differentiated biotinized antigen-specific antibodies CD45R, CD4, CD8, Gr-1, Ter119, CD11c (all from Pharmingen) as differentiation markers (Lin), and then the admixture was allowed to stand for 20 minutes in ice to react. After washing 2 times with staining buffer, cells simultaneously CD34-negative, Sca-1-positive, c-Kit-positive, and Lin-negative were fractionated using a cell sorter (FACS Vantage, Becton Dickinson).

### Example 2: Liquid culture of cytokine cocktail and murine hematopoietic stem cells

Murine hematopoietic stem cells prepared as described in Example 1 were seeded in a 96 well U-bottom plate at 50 cells/well and cultured. A medium used for the culture was S-clone SF03 (Sanko Pure Chemicals) supplemented with 0.01% BSA. As a cytokine cocktail, 50 ng/ml each of murine SCF (Kirin Brewery), human IL-6 (Kirin Brewery), murine IL-11 (R&D Systems), and Flt-3 ligand (R&D Systems), or 50 ng/ml each of murine SCF (Kirin Brewery), FP6 (Tosoh Corp.), and Fit-3 ligand (R&D Systems) were added, and cultivation was carried out at 37°C for 7 days in an atmosphere of 5% CO₂. After cultivating for 7 days, a competitive long term hematopoietic reconstitution assay was carried out as described in Example 3 to determine the rate of hematopoietic stem cell/hematopoietic progenitor cell expansion.

### Example 3: Transplantation of hematopoietic cells into irradiated mice

The cells cultured in Example 2 and Example 3 together with 200,000 each of whole bone marrow cells (derived from C57BL/6-Ly5.2 mouse, Charles River) were transplanted into 5 per group of C57BL/6-Ly5.2 mice (8-week-old males, Charles River) irradiated with an 8.5 Gy dose of X ray, via tail vein injection. After the transplantation, peripheral blood was collected with time from the eye socket and the rate of the number of cells derived from C57BL/6-Ly5.1 pep mice was calculated by FACS. Peripheral blood was analyzed by an ordinary method (Basic Technology in Immunological Research by Kiyoshi Takatsu. Yodosha, 1995). Hemolysis was carried out by adding 350 µl of distilled water to 50 µl of peripheral blood and allowing the admixture to stand for 30 seconds, after which 2-fold concentrated PBS was added and leukocytes were recovered by centrifugation. The cells were washed once with staining buffer (PBS containing 5% FCS and 0.05% NaN₃), after which anti-CD16 antibody, FITC-binding anti-Ly5.1 (CD45.1) antibody, phycoerythrin-binding anti-Gr-1 and CD11c antibodies, and allophycocyanin-binding CD90 (Thy1) and CD45R (B220) antibodies (all from Pharmingen) were added and the admixture was allowed to stand for 30 minutes in ice to react. Next, after washing with staining buffer, FACS analysis was carried out. After the transplantation, the rate of Ly5.1 positive in Gr-1- or CD11c-positive cells (myelocytic lineage) and the rate of Ly5.1 positive in CD90- or CD45R-positive cells (lymphocytic lineage) in the peripheral blood were each calculated with time to estimate the increase or decrease in the number of cells capable of reconstitution during the hematopoietic stem cell cultivation.

As shown in Figure 1, it is possible to significantly increase hematopoietic progenitor cells capable of short-term reconstitution of highly chimeric bone marrow 2 weeks after the transplantation, by adding cytokines to the culture. However, from a month downward chimerism of the cells cultured in the presence of cytokines rapidly decreased to the level lower than chimerism of stem cells transplanted without cultivation; which indicates that the hematopoietic stem cells differentiated and lost their characteristic ability for long-term reconstitution during the culture. On the other hand, comparing the cytokine combination of SCF + FP6 + FL with the cytokine combination of SCF + IL-6 + IL-11 + FL, the former maintained higher chimerism over a long period of time. This result indicates that expansion of hematopoietic cells in the culture system with FP6 is different from those with IL-6 and IL-11 that similarly transduce gp130 receptor signal. The former exhibited higher chimerism than the latter even 3 months after the transplantation, showing that the cytokine combination containing FP6 can expand more undifferentiated hematopoietic cells.

### Example 4: Cocultivation of murine AGM-S3-A9 stromal cells with hematopoietic stem cells

AGM-S3 stromal cells were prepared according to WO99/03980 or Japanese Patent Laid-Open Publication No. 37471/2001, and the obtained AGM-S3 cells were subcloned to obtain AGM-S3-A9 cells as subclone cells highly active in supporting hematopoietic stem cells derived from human umbilical cord blood, namely subclone cells highly active in expanding or maintaining BFU-E. AGM-S3-A9 cells (cultured in an MEMα medium containing 10% FCS) were seeded onto a 24-well culture plate at 1 × 10⁵/well and cultured for 1 day until the cells were grown to confluence at the bottom of the wells. Then, the medium was replaced with 1 ml of fresh medium (MEMα medium supplemented with 10% FCS) containing 20 ng/ml each of SCF (Kirin Brewery) and TPO (Kirin Brewery) and 20 ng/ml or 50 ng/ml of FP6 (Tosoh Corp.), 100 murine hematopoietic stem cells (derived from C57BL/6-Ly5.1) obtained in Example 1 were overlaid on these cells, and then cultivation was restarted. On day 7 of cultivation, cells were treated with trypsin (allowed to stand at 37°C for 2 to 5 minutes with PBS containing 0.05% trypsin and 0.5 mM EDTA) , all the cells including stromal cells in the culture plate were recovered and cells corresponding to one half of the culture system were transplanted into one mouse. As a control, a culture system (an MEMα medium supplemented with 10% FCS) containing only a cytokine cocktail (each cytokine concentration was 20 ng/ml) was simultaneously prepared and recovered as described in Example 2. The state of growth of the hematopoietic stem cells or hematopoietic progenitor cells was analyzed using the recovered cells as described in Example 3 (Figure 2).

In the liquid culture only with SCF + TPO + FP6, high chimerism similar to that in Example 3 was observed 2 weeks after the transplantation, but the chimerism decreased over the period of 2 months after the transplantation. On the other hand, in cocultivation of AGM-S3-A9 stromal cells with SCF + TPO + FP6, high chimerism similar to that in cultivation only with cytokine was observed 2 weeks after the transplantation and the high chimerism exceeding to that in input was maintained beyond 2 months after the transplantation. The increased chimerism highly exceeding that in input shows that hematopoietic stem cells were present more at the end of the cocultivation of AGM-S3-A9 stromal cells with SCF + TPO + FP6 than at the start of the cultivation, indicating that the hematopoietic stem cells were self-renewed to expand in this culture system. This result shows that not only hematopoietic progenitor cells but also hematopoietic stem cells can be expanded when hematopoietic stem cells are cultured in the presence of AGM-S3-A9 stromal cells and SCF + TPO + FP6.

### Example 5: Cocultivation of human mesenchymal stem cells (hMSCs) with hematopoietic stem cells

Human mesenchymal stem cells (hMSCs) (CAMBREX, cat#: PT-2501, lot#: 0F0558, cultured in a specified medium: hMSC BulletKit) were seeded onto a 24-well culture plate at 1 × 10⁵/well and cultured for 1 day until the cells were grown to confluence at the bottom of the wells. Then, the medium was replaced with 1 ml of fresh medium (HEMα medium supplemented with 10% FCS) containing 20 ng/ml each of SCF (Kirin Brewery), TPO (Kirin Brewery) and FP6 (Tosoh Corp.), 50 murine hematopoietic stem cells (derived from C57BL/6-Ly5.1) obtained in Example 1 were overlaid on these cells, and cultivation was restarted. On day 7 of cultivation, cells were treated with trypsin (allowed to stand at 37°C for 2-5 minutes with PBS containing 0.05% trypsin and 0.5 mM EDTA), and all the cells including stromal cells on the culture plate were recovered. As a control, a coculture system with hMSCs without addition of a cytokine cocktail was prepared and cells were simultaneously recovered. The state of growth of the hematopoietic stem cells was analyzed using the recovered cells by the method described in Example 3 (Fig. 3).

In the cocultivation with hMSCs alone, no increase in chimerism was observed and both hematopoietic progenitor cells and hematopoietic stem cells disappeared during the cultivation. On the other hand, in cocultivation of hMSCs and hematopoietic stem cells in the presence of the cytokine cocktail, SCF + TPO + FP6, high chimerism was observed 2 weeks after the transplantation and furthermore, the high chimerism exceeding that in input was maintained even beyond 2 months after the transplantation. This increased chimerism highly exceeding that in input shows that hematopoietic stem cells were present more at the end of the cocultivation of hMSCs with SCF + TPO + FP6 than at the start of cultivation, indicating that the hematopoietic stem cells were self-renewed to expand in this culture system. This result shows that not only hematopoietic progenitor cells but also hematopoietic stem cells can be expanded when hematopoietic stem cells are cultured in the presence of hMSCs and SCF + TPO + FP6.

### Example 6: Evaluation of activity for supporting human hematopoietic stem cells and hematopoietic progenitor cells

### (1) Isolation of human umbilical cord blood CD34-positive stem cells

Human umbilical cord blood was collected upon normal delivery in accordance with the Standard of Research Ethics Committee of Kirin Brewery Pharmaceutical Research Laboratories. Umbilical cord blood was collected using a syringe with heparin (Novo Nordisk) added to prevent clotting. The heparinised umbilical cord blood was overlaid on Ficoll-paque (Pharmacia) and mononuclear cells were separated using density gradient centrifugation (400 × g, at room temperature for 30 minutes). Erythrocytes contaminating in the mononuclear cell fraction were lysed by treating with an erythrocyte-lysing agent (PharmLyse™, Pharmingen) at room temperature for 5 minutes. The mononuclear cells were washed with PBS containing 1 mM EDTA and then allowed to stand for 30 minutes under ice-cold conditions with addition of CD34 antibody-binding magnetic beads (Direct CD34 isolation kit, Miltenyi Biotec). After washing the cells, cells expressing CD34 were separated using a magnetic column (MidiMACS, Miltenyi Biotec). This cell population was used as a human umbilical blood-derived hematopoietic stem cell population.

### (2) Cocultivation of human hematopoietic stem cells with AGM-S3 subclone (AGM-S3-A9) cells

On the previous day, A9 cells were seeded at 2 × 10⁵/well (6-well culture plate, Falcon), and cultured in 1 ml of an MEMα medium containing 10% FCS until the cells were grown to the confluence at the bottom of the well. The above-mentioned stromal cells were overlaid with human umbilical cord blood-derived CD34-positive hematopoietic stem cells at 10,000 cells/well and cocultivation was carried out with 4 ml of an HEMα medium supplemented with 10% FCS with addition of a cytokine cocktail (STF + FP6) containing 20 ng/ml FP6, 20 ng/ml human SCF, 20 ng/ml human TPO (Kirin Brewery), and 100 ng/ml human FL (R&D Systems, catalogue No. 308-FK)). In control groups to compare growth activity, cocultivation was carried out with A9 cells alone in a medium without cytokines, or alternatively, cultivation was carried out in the absence of A9 cells in 2 ml of the medium containing the above-mentioned cytokine cocktail using a 12-well plate. One week after the start of cocultivation, one half of the medium was removed and 1 ml of fresh medium was added. Two weeks after the start of cocultivation, stromal cells and human blood cells were treated with trypsin (allowed to stand at 37°C for 5 to 10 minutes with PBS containing 0.05% trypsin and 0.5 mM EDTA (GIBCO BRL)) to simultaneously detach them from the culture plate, and subjected to colony assay to analyze the growth of hematopoietic stem cells or hematopoietic progenitor cells.

### (3) Evaluation of the state of growth of hematopoietic stem cells and hematopoietic progenitor cells by colony assay

Cells grown in the abovementioned cocultivation systems were appropriately diluted and transferred to 1 ml of a methylcellulose culture system and analyzed in quadruplicate. Further, as a control to compare expansion effect, a colony assay was similarly carried out with CD34-positive cells before cocultivation. In the methylcellulose culture system, cultivation was carried out in a 35-mm petri dish (Nunc) using an IMDM medium (GIBCO BRL) containing 0.89% methylcellulose (Shin-Etsu Chemical Co.) supplemented with 10% fetal calf serum (Hyclone), 2 mM L-glutamine (GIBCO BRL), 1 mM sodium pyruvate (Wako Chemicals), 0.5 µM 2-mercaptoethanol and antibiotics (final concentration of 100 U/ml penicillin, 100 µg/ml streptomycin, and 250 ng/ml amphotericin B (antibiotic-antimycotic (×100), liquid, GIBCO BRL) with addition of 100 ng/ml each of human SCF and human IL-6, 10 ng/ml each of human IL-3, human G-CSF, and human TPO, and 4 IU/ml EPO (all from Kirin Brewery), as cytokines. The above-mentioned various hematopoietic factors are all purified recombinants. After cultivation for 2 weeks, colonies formed were observed under a microscope and counts were made for CFU-GM (granulocyte-macrophage colony-forming unit), BFU-E (erythroid burst forming unit), and CFU-Emix (erythrocyte mixed colony-forming unit).

Figure 4 shows the result of studying the state of expansion of hematopoietic stem cells or hematopoietic progenitor cells after 2-week cultivation of CD34-positive hematopoietic stem cells in the presence or absence of AGM-S3-A9 stromal cells. By using the AGM-S3-A9 stromal cells and cytokine cocktail medium, expansion of all of the differentiation lineage cells, CFU-GM, BFU-E, and CFU-Emix, was supported better than that before cocultivation. Further, comparison of the culture system with cytokines alone and that with addition of the stromal cells showed that the presence of the stromal cells in the culture system expanded all colonies. In particular, more expansion of erythrocyte precursor cells, i.e., BFU-E and CFU-E mix, which are generally known to be difficult to be sustained, was observed in the coculture system with addition of stromal cells than in the culture system with cytokines alone. Thus, also for human hematopoietic cells, it becomes possible to enhance expansion of hematopoietic stem cells or hematopoietic progenitor cells by adding a cytokine cocktail including sIL-6R-IL-6 fusion protein in the presence of stromal cells.

### (4) Effect of FP6 on hematopoietic stem cell supporting activity by A9 and cytokine cocktail

FP6 activity in the hematopoietic stem cell growth culture system of the present invention was evaluated. Evaluation was carried out according to the system described in (2) and (3) above, in which stem cell supporting ability was compared using human umbilical cord blood-derived CD34-positive hematopoietic stem cells in AGM-S3-A9 coculture system with addition of a cytokine cocktail with FP6 (SCF + TPO + FL + FP6) or a cytokine cocktail without FP6 (SCF + TPO + FL), or non-coculture system, using CFU-GM, BFU-E, and CFU-Emix as indexes. Figure 5 shows the result of cocultivation for 2 weeks. The presence of stromal cells enabled all of the colony forming cells to expand in the system with either cytokine cocktails. When cytokine combinations of SCF + TPO + FL + FP6 and SCF + TPO + FL were compared, expansion of more colony forming cells was confirmed in the combination with FP6.

It has already been reported that SCID reconstituting cells can be effectively expanded by adding sIL-6R-IL-6 fusion protein to SCF + TPO + FL, or adding IL-6R and IL-6 to SCF + TPO + FL in the absence of stromal cells in a culture system (Kollet O, Aviram R, Chebath J, ben-Hur H, Nagler A, Shultz L, Revel M, Lapidot T. sIL-6R-IL-6: The soluble interleukin-6 (IL-6) receptor/IL-6 fusion protein enhances in vitro maintenance and proliferation of human CD34 (+) CD38 (-/low) cells capable of repopulating severe combined immunodeficiency mice, Blood 1999 Aug 1 94:3 923-31; Ueda T, Tsuji K, Yoshino H, Ebihara Y, Yagasaki H, Hisakawa H, Mitsui T, Manabe A, Tanaka R, Kobayashi K, Ito M, Yasukawa K, Nakahata T. Expansion of human NOD/SCID-repopulating cells by stem cell factor, Flk2/Flt3 ligand, thrombopoietin, IL-6, and soluble IL-6 receptor. J Clin Invest. 2000 Apr 105(7):1013-21). However, in the present Example, more expansion of colony forming cells was confirmed in the system with addition of stromal cells to the above-mentioned cytokine combination than in the system with the cytokine cocktail alone. The result of the present Example shows that the presence of stromal cells enables more colony forming cells to expand in a culture system as compared to a cytokine cocktail alone, indicating that human hematopoietic stem cells are being expanded.

TPO activity in the hematopoietic stem cell culture system of the present invention was studied. Evaluation was carried out according to the system described in (2) and (3) above, in which stem cell supporting ability was compared using human umbilical cord blood-derived CD34-positive hematopoietic stem cells in AGM-S3-A9 coculture system with addition of a cytokine cocktail with TPO (SCF + TPO + FL. + FP6) or a cytokine. cocktail without TPO (SCF + FL + FP6), using CFU-GM, BFU-E, and CFU-Emix as indexes. Figure 6 shows the result. Expanding effect observed was 3.8 times for BFU-E and 33 times for CFU-Emix higher in the system with TPO (SCF + TPO + FL + FP6) than in the system without TPO (SCF + FL + FP6). This result shows that TPO is selected as a cytokine to be added to the present hematopoietic stem cell culture system. Further, it shows that cytokines suitable for the present culture system can be selected in the same manner as described above.

## Claims

1. A process for producing hematopoietic stem cells comprising the step of culturing hematopoietic stem cells in the presence of a gp130 stimulating factor, one or more cytokines, and stromal cells.

2. The process according to claim 1, wherein the gp130 stimulating factor is a fusion protein of interleukin-6 (IL-6) or its mutant with IL-6 receptor α-chain (IL-6R) or its mutant, a combination of IL-6R or its mutant and IL-6 or its mutant, an antibody to gp130 which acts as an agonist to gp130, interleukin-11 (IL-11) , leukocyte migration inhibitory factor (LIF), oncostatin M, or cardiotropin.

3. The process according to claim 1, wherein the gp130 stimulating factor is a fusion protein of IL-6 or its mutant and IL-6R or its mutant.

4. The process according to claim 3, wherein the fusion protein is a protein (FP6) comprising a fragment from residue 112 to residue 333 of IL-6R directly linked at its C-terminal to the N-terminal of a fragment from residue 38 to residue 212 of IL-6.

5. The process according to claim 1, wherein the cytokines are factors that expand and/or maintain hematopoietic stem cells.

6. The process according to claim 1, wherein the cytokines are stem cell factor (SCF), thrombopoietin (TPO) or its mutants or their derivatives, Flt-3 ligand (FL), or combinations thereof.

7. The process according to claim 1, wherein the cytokines comprise at least thrombopoietin (TPO) or its mutants or their derivatives.

8. The process according to claim 1, wherein the stromal cells are AGM region-derived cells, bone marrow-derived stromal cells, mesenchymal stem cells, fibroblasts, vascular endothelial cells, fetal liver-derived cells, or preadipocytes.

9. The process according to any one of claims 1 to 8, further comprising the step of culturing stromal cells prior to the step of culturing hematopoietic stem cells.

10. A method of culturing hematopoietic stem cells comprising the step of culturing hematopoietic stem cells in the presence of a gp130 stimulating factor, one or more cytokines, and stromal cells.

11. A method of producing hematopoietic stem cells comprising the step of culturing hematopoietic stem cells in the presence of a fusion protein of interleukin-6 (IL-6) and IL-6 receptor a-chain (IL-6R); thrombopoietin (TPO); and stromal cells selected from AGM region-derived cells, bone marrow-derived stromal cells, and mesenchymal stem cells; and optionally, a stem cell factor (SCF) and an Flt-3 ligand (FL).

12. A medium used for culturing hematopoietic stem cells, comprising a gp130 stimulating factor, one or more cytokines, and stromal cells.

13. A medium used for culturing hematopoietic stem cell, comprising a fusion protein of interleukin-6 (IL-6) and IL-6 receptor α-chain (IL-6R); thrombopoietin (TPO); and stromal cells selected from AGM region-derived cells, bone marrow-derived stromal cells; and mesenchymal stem cell; and optionally, a stem cell factor (SCF) and an Flt-3 ligand (FL).
